# EUROPEAN PATENT SPECIFICATION

(11) **EP 3 229 718 B1**
(45) Date of publication and mention of the grant of the patent: **27.02.2019**
(21) Application number: 15739570.8
(22) Date of filing: 16.07.2015
(51) Int. Cl.: A61B 18/12, A61B 17/32, A61B 18/14

(54) **A COMBINED ULTRASONIC AND HF SURGICAL SYSTEM AS WELL AS A CONTROL DEVICE AND A METHOD THEREOF**
CHIRURGISCHES ULTRASCHALL- UND HF-KOMBINATIONSSYSTEM SOWIE STEUERUNGSVORRICHTUNG UND VERFAHREN DAFÜR
SYSTÈME CHIRURGICAL HF ET ULTRASONORE COMBINÉ, DISPOSITIF DE COMMANDE ET SON PROCÉDÉ

(30) Priority: 08.12.2014 DE 102014225178
(43) Date of publication of application: 18.10.2017
(73) Proprietor: Olympus Corporation, Hachioji-shi Tokyo 192-8507 (JP)
(72) Inventor: NOACK, Andreas, 21423 Drage (DE); AKAGANE, Tsunetaka, Hachioji-shi Tokyo 192-0023 (JP)
(74) Representative: Eisenführ Speiser
(86) International application number: PCT/EP2015/066362
(87) International publication number: WO 2016/091400

(56) References cited:
- JP-A- 2000 271 145
- US-A1- 2010 168 742
- US-A1- 2012 078 139

## Description

The invention relates to a combined ultrasonic and HF surgical system (HF: High Frequency), comprising an ultrasonic generator having a generation unit for providing an excitation signal, by which an ultrasonic converter can generate an ultrasonic vibration, and a HF generator for providing a HF energy at two output contacts. The term "high frequency" is understood herein and in the following as electromagnetic waves in the range of radio frequency waves. Therefore, both terms HF and radiofrequency (RF) can be used interchangeably.

The invention further relates to a control device for controlling such a combined ultrasonic and HF surgical system as well as a method for operating such a combined ultrasonic and HF surgical system.

Combined ultrasonic and HF surgical systems and instruments thereof are known, for example from DE 100 21 529 A1, US 6,328,703 D1 and DE 10 2009 041 329 A1. In the known systems and instruments, ultrasonic energy and HF energy may be applied simultaneously or sequentially. Instruments for such systems are known in which an applicator can be supplied for example simultaneously with HF energy and ultrasonic energy.

The existing systems and instruments provide several advantages, however further enhancements are desirable.

Thus, it is an object of the present invention to provide a combined ultrasonic and HF surgical system, a control device for such a system as well as a method for controlling and/or operating such a system, which are enhanced compared to existing systems, control devices and methods.

This object is accomplished by a combined ultrasonic and HF surgical system, comprising an ultrasonic generator having a generation unit for providing an excitation signal by which an ultrasonic converter can generate an ultrasonic vibration, a HF generator for providing a HF energy at two output contacts, and a control unit adapted to control the ultrasonic generator and/or the HF generator, such that the excitation signal and the HF energy are provided in a coordinated fashion, such that the HF energy is provided in dependency of the excitation, wherein a HF energy is modulated with the frequency of the excitation signal or with a harmonic of that frequency.

The combined ultrasonic and HF surgical system according to the present invention is based on the finding that medical devices which use combined ultrasonic and HF energy have superior coagulating ability and fast cutting ability, even if comparatively low energies are used. It is currently believed that the reason for this is that the ultrasonic energy lowers the saturated vapor pressure intermittently around the ultrasonic probe. The invention is further based on the finding that enhancements may be achieved, if the provision of ultrasonic and HF energy not only takes place simultaneously but coordinated. A coordinated provision can be understood in a way that the HF energy is provided in dependency of the excitation signal of the ultrasonic generator and/or an ultrasonic vibration generated by means of an ultrasonic converter. Such a coordinated provision allows for example for adjusting and/or alternating the amplitude and/or the frequency of the HF energy. By the control of the ultrasonic generator and/or the HF energy, such that the HF energy is provided in dependency of the excitation signal and/or the ultrasonic vibration, the utilization of the combined ultrasonic and HF surgical system may be expanded and/or, even more detailed, adapted to different situations of usage. In particular, by the coordination of the ultrasonic and HF energy also synergetic effects may be achieved in the operation of the combined ultrasonic and HF surgical system.

The HF generator preferably comprises a radiofrequency oscillator. The HF energy is usually provided at two output contacts of a HF generator, which may be for example connected to a bipolar electrosurgical instrument. The high frequent alternating current generated by the HF generator may have a frequency in the range of 0.2 - 3 MHz.

Preferably, the ultrasonic generator comprises an ultrasonic frequency oscillator. In operation, the ultrasonic generator may generate an alternating current having a frequency of about 20 - 50 KHz. This excitation signal may be converted into an ultrasonic vibration by means of an ultrasonic converter. Such ultrasonic converter is usually located in an instrument connected to the ultrasonic generator. In the instrument, the ultrasonic vibration is further transferred to an ultrasonic applicator, by means of which the ultrasonic vibration may be introduced into the tissue to be treated. The instrument or an applicator of the instrument, respectively, may be driven for example to an ultrasonic vibration which is substantially directed in axial direction of a shaft of the instrument or in a direction substantially perpendicular to the instrument shaft or on a very small circular arc section, respectively. The instrument can for example be utilized by a user, such that the ultrasonic vibration takes place substantially perpendicular to a tissue surface (press or push movements, respectively) or substantially parallel to a tissue surface (slide or cutting movements, respectively).

In an advantageous manner a combined instrument is utilized, by which the ultrasonic vibration as well as the HF energy can be introduced into the tissue. For example, the ultrasonic vibration can be used for cutting of tissue, while the HF energy can be used for coagulating the tissue to stop bleeding.

The control unit according to the present invention is adapted and arranged to control the ultrasonic generator and/or the HF generator. Preferably, main operational parameters of the radiofrequency oscillator and the ultrasonic frequency oscillator are set by the control unit based on stored operational programs and/or user input through a graphical user interface. The control unit may have an ultrasonic control unit, which is adapted to control a generation unit of an ultrasonic generator, such that an excitation signal is provided by which an ultrasonic converter can generate an ultrasonic vibration. The control unit may further have a HF control unit, which is adapted to control a HF generator such that HF energy is provided at two output contacts of the HF generator. The control unit may further have a synchronization unit which carries out the coordination of the provision of excitation signal or ultrasonic energy, respectively, and HF energy. The ultrasonic control unit, HF control unit and/or synchronization unit may be separate units or one integral unit. If the control unit is described herein, this comprises embodiments having separate units for ultrasonic control, HF control and combined control for coordination of ultrasonic and HF energy as well as one integral unit thereof.

The combined ultrasonic and HF surgical system can preferably be operated in different operating modes which may be selected by a user by means of an input unit. Such operating modes may be an isolated ultrasonic operation, an isolated HF surgical operation or a combined ultrasonic and HF surgical operation. Different methods of combined, coordinated control of ultrasonic and HF energy described further below may be embodied in preset operation modes selectable by a user. Also the vibration direction of the instrument or the ultrasonic applicator of the instrument, respectively, and/or the desired kind of usage (press or push movements, respectively, or slide or cut movements, respectively) may preferably be selected and/or (pre)set, as operation modes at the combined ultrasonic and HF surgical system. The control parameters for different operating modes are preferably stored in the combined ultrasonic and HF surgical system, for example in the control unit.

HF energy may be provided with predetermined parameters, in particular parameters relating to duration, frequency, amplitude and/or output power of the HF signal. These parameters may be selected or set by a user and/or they may be preset or preselected in different operating modes.

According to the present invention, the control unit is adapted to control the ultrasonic generator and/or the HF generator, such that HF energy is modulated with the frequency of the excitation signal. This feature of the combined ultrasonic and HF surgical system according to the present invention is of particular advantage, if the ultrasonic vibration is applied by an instrument, which is connected to the combined ultrasonic and HF surgical system, substantially perpendicular to the tissue surface in the sense of a push or press movement, respectively.

The modulation of the HF energy with the frequency of the excitation signal may be implemented such that a HF energy is provided with a first amplitude and/or with a first frequency at a time when the ultrasonic applicator of an instrument exerts a high pressure onto the tissue and that HF energy is provided with a second amplitude, which is smaller than the first amplitude, and/or with a second frequency, which is smaller than the first frequency, at a time when an ultrasonic applicator of an instrument exerts only a low or no pressure onto the tissue. In this way, for example a penetration of the ultrasonic applicator of an instrument is supported by the HF energy.

The second amplitude may preferably be at least 25% less, or more preferably 50% less than the first amplitude. The second amplitude may even be zero amplitude.

The second frequency may preferably be at least 10% less, or more preferably 20% less than the first frequency.

According to a preferred embodiment of the combined ultrasonic and HF surgical system, the control unit comprises a synchronization unit which is adapted to receive the excitation signal from the ultrasonic generator, to generate at least one synchronized modulation signal in dependency of the excitation signal, and to output the modulation signal to the HF generator.

Preferably, the modulation signal is received by the radiofrequency oscillator of the HF generator and processed accordingly. Further preferably, a first synchronized modulation signal may be used to effect amplitude modulation of the HF energy signal. A second synchronized modulation signal may be used to effect frequency modulation of the HF energy signal. Synchronized modulation signals may be generated with the frequency of the ultrasonic frequency oscillator, or with a harmonic of that frequency, e.g. double or treble frequency, as regulated by the control unit based on stored operational programs and/or user input through a user interface.

According to a further preferred embodiment of the combined ultrasonic and HF surgical system, the control unit is adapted to control the ultrasonic generator and/or the HF generator, such that a HF energy is modulated with a harmonic, in particular with the double or treble frequency of the excitation signal. This embodiment may particularly be useful if the ultrasonic vibration is applied substantially parallel to a tissue surface in the sense of a slide or cut movement, respectively. The modulation of the HF energy with twice the ultrasonic frequency may be utilized advantageously to provide HF energy with a first amplitude and/or with a first frequency at a time when the moving velocity of the ultrasonic applicator of the instrument is low or equal to zero and to provide HF energy with a second amplitude, which is smaller than the first amplitude, and/or with a second frequency, which is smaller than the first frequency, at a time when the moving velocity of the ultrasonic applicator is high. Thereby, a cutting movement can for example be supported by the HF energy.

In a further embodiment of the combined ultrasonic and HF surgical system the control unit is adapted to control the ultrasonic generator and/or the HF generator, such that a HF energy is provided with a first amplitude and/or with a first frequency at a time when an instrument, which is moved by the ultrasonic vibration, and/or the excitation signal is at a turning point, and that HF energy is provided with a second amplitude, which is smaller than the first amplitude, and/or with a second frequency, which is smaller than the first frequency, at a time when the instrument, which is moved by the ultrasonic vibration, and/or the excitation signal is between two turning points.

The term "turning point" is understood herein as the point at which the moving direction of an instrument moved by the ultrasonic vibration turns and/or the excitation signal reaches a maximum (positive or negative). There may however be a phase shift between the turning point of the excitation signal and the turning point of the instrument due to resonance effects. Preferably, the HF energy is to be provided with a first amplitude and/or with a first frequency in particular at a time when the instrument, moved by the ultrasonic vibration, and/or the excitation signal is at such a turning point or in a region of such a turning point. The region of such a turning point can for example comprise 10%, 20%, 25%, 30%, 40% or 50% of the curve between the turning point and the zero crossing or the next turning point to both sides of the turning point.

In a further preferred embodiment, the control unit is adapted to control the ultrasonic generator and/or the HF generator, such that HF energy is provided with a first amplitude and/or with a first frequency at a time when an instrument, which is moved by the ultrasonic vibration, and/or the excitation signal is between two turning points, and such that HF energy is provided with a second amplitude, which is smaller than the first amplitude, and/or with a second frequency, which is smaller than the first frequency, at a time when the instrument, which is moved by the ultrasonic vibration and/or the excitation signal is at a turning point.

This embodiment illustrates a quasi-inversion of the alternative described above: In the embodiment described herein a HF energy is provided with a first amplitude and/or with a first frequency at a time when the instrument, which is moved by the ultrasonic vibration, and/or the excitation signal is between two adjacent turning points or between the regions of two adjacent turning points, respectively, which - with reference to the excitation signal - can also be indicated as a section around the zero crossing. In contrast to this, HF energy is provided with a second amplitude, which is smaller than the first amplitude, and/or with a second frequency, which is smaller than the first frequency, at a time when the instrument, which is moved by the ultrasonic vibration, and/or the excitation signal is at a turning point or in a region of a turning point, respectively.

According to a further preferred embodiment of a combined ultrasonic and HF surgical system, the control unit is adapted to control the ultrasonic generator and/or the HF generator, such that HF energy is provided with a first amplitude and/or with a first frequency at a time when an instrument, which is moved by the ultrasonic vibration, and/or the excitation signal is at a first turning point or in a region of a first turning point, respectively, and that HF energy is provided with a second amplitude, which is smaller than the first amplitude, and/or with a second frequency, which is smaller than the first frequency, at a time when the instrument, which is moved by the ultrasonic vibration, and/or the excitation signal is at a second turning point or in a region of the second turning point, respectively, or between two adjacent turning points or between the regions of two adjacent turning points, respectively.

In this embodiment, the HF energy is provided with a first amplitude and/or with a first frequency only when the instrument, which is moved by the ultrasonic vibration, and/or the excitation signal is at a first turning point or in the region of the first turning point, respectively, whereas at a second turning point and in the region between the two turning points HF energy is provided with a second amplitude, which is smaller than the first amplitude, and/or with a second frequency, which is smaller than the first frequency. The first turning point can be for example a maximum turning point, while the second turning point can be a minimal turning point of the excitation signal, or the other way around.

A further preferred embodiment of the combined ultrasonic and HF surgical system is characterized in that the control unit is adapted to control the ultrasonic generator and/or the HF generator such that HF energy is provided with a first amplitude and/or with a first frequency at a time when an instrument, which is moved by the ultrasonic vibration, has no or a low moving velocity, and that HF energy is provided with a second amplitude, which is smaller than the first amplitude, and/or with a second frequency, which is smaller than the first frequency, at a time when the instrument, which is moved by the ultrasonic vibration, has a high moving velocity. In this embodiment, HF energy is provided with a first amplitude and/or with a first frequency at a time when the instrument has no or a low moving velocity due to the ultrasonic vibration, i.e. for example when it changes its moving direction. However, while the instrument has a high moving velocity due to the ultrasonic vibration, HF energy is provided with a second amplitude, which is smaller than the first amplitude, and/or with a second frequency, which is smaller than the first frequency.

The term "high moving velocity" of the instrument is understood as a moving velocity due to the ultrasonic vibration which is at least one half or at least three quarters of the maximum moving velocity occurring in the operation mode. The term "low moving velocity" of the instrument is understood as a moving velocity due to the ultrasonic vibration of the instrument, which is lower than one half or one quarter of the maximum moving velocity in the respective operating mode (low moving velocity).

In a further preferred embodiment the control unit is adapted to control the ultrasonic generator and/or the HF generator such that HF energy is provided with a first amplitude and/or with a first frequency at a time when an instrument which is moved by the ultrasonic vibration has a high moving velocity, and that HF energy is provided with a second amplitude, which is smaller than the first amplitude, and/or with a second frequency, which is smaller than the first frequency, at a time when the instrument, which is moved by the ultrasonic vibration has a lower or no moving velocity.

This embodiment illustrates an inversion of the preceding embodiment: In the embodiment described herein, a high moving velocity due to the ultrasonic vibration of the instrument is supported by HF energy with a first amplitude and/or with a first frequency, whereas HF energy is provided with a second amplitude, which is smaller than the first amplitude, and/or with a second frequency, which is smaller than the first frequency, when the moving velocity due to the ultrasonic vibration of the instrument is low or equal to zero, for example in a change of direction.

According to a further preferred embodiment, the control unit is adapted to control the ultrasonic generator and/or the HF generator such that HF energy is provided with a first amplitude and/or with a first frequency at a time when an instrument, which is moved by the ultrasonic vibration, accelerates its moving velocity, and that HF energy is provided with a second amplitude, which is smaller than the first amplitude, and/or with a second frequency, which is smaller than the first frequency, at a time when an instrument, which is moved by the ultrasonic vibration, decelerates its moving velocity. The time when the instrument accelerates its moving velocity is preferably understood as a phase starting after a turning point and substantially ending half way to an adjacent turning point.

While moving through the tissue, the instrument exerts a pressure onto the tissue. During the phase of acceleration, this pressure is increased. While the pressure is increased, the power of the HF energy is provided or the power of the HF energy is increased, respectively. Therefore, this embodiment provides the advantage that coagulating ability is improved in the phase of acceleration.

In another preferred embodiment the control unit is adapted to control the ultrasonic generator and/or the HF generator such that a HF energy is provided with a first amplitude and/or with a first frequency at a time when an instrument, which is moved by the ultrasonic vibration, decelerates its moving velocity, and that HF energy is provided with a second amplitude, which is smaller than the first amplitude, and/or with a second frequency, which is smaller than the first frequency, at a time when an instrument, which is moved by the ultrasonic vibration, accelerates its moving velocity. The time when the instrument decelerates its moving velocity is preferably understood as a phase which ends at a turning point und starts substantially half way from an adjacent preceding turning point.

While moving through the tissue, the instrument exerts a pressure onto the tissue. During the phase of decelaration, this pressure is decreased. While the pressure is decreased, the power of the HF energy is provided or the power of the HF energy is increased, respectively. Therefore, this embodiment provides the advantage that the cutting ability is improved in the phase of deceleration.

A further preferred embodiment of the combined ultrasonic and HF surgical system according to the present invention is characterized in that the modulation of a HF energy is executed as an amplitude modulation by
- modifying the activation time of a switch provided for feeding a resonant circuit, or
- cyclically switching the HF energy substantially on and off.

A further preferred embodiment of the combined ultrasonic and HF surgical system according to the present invention is characterized in that the modulation of a HF energy is executed as a frequency modulation by
- varying a capacity included in a resonant circuit for frequency modulation, or
- switching between at least two operating frequencies.

Modulation of the radiofrequency oscillator may be effected by various techniques, such as modifying the activation time of energizing switches for feeding a resonant circuit to achieve amplitude modulation, or by varying a capacity included in a resonant circuit to achieve frequency modulation. The resonant circuit may preferably include an inductor-capacitor circuit (also called LC circuit). Modulation may also be effected in the form of discrete switching between two operating frequencies or by cyclically switching the radiofrequency treatment signal on and off. Instead of switching the treatment signal off completely, it may be switched to a very low amplitude which has no therapeutic effect, but enables the radiofrequency oscillator to maintain in resonant operation.

The different embodiments may offer different advantages for different tissues and/or different applications. It is therefore particularly preferred if a combined ultrasonic and HF surgical system, or the control unit of such combined ultrasonic and HF surgical system, is adapted to implement at least two, or a number of, preferably all the embodiments described above, for which different preset operating modes may be provided, which can be selected by a user by means of a user interface, for example a graphical user interface comprising a touch screen.

According to a further aspect of the invention there is provided a control device for controlling a combined ultrasonic and HF surgical system, as described above, comprising a control unit adapted to control an ultrasonic generator and/or a HF generator, such that an excitation signal, by which an ultrasonic converter can generate an ultrasonic vibration, and the HF energy are provided in a coordinated fashion such that the HF energy is provided in dependency of the excitation signal, wherein the HF energy is modulated with the frequency of the excitation signal or with a harmonic of that frequency.

The control device according to the present invention and possible enhancements thereof have features which make them in particular suitable to be used for a combined ultrasonic and HF surgical system according to the present invention. With regard to advantages, embodiments as well as embodiment details of the control device and its enhancements it is referred to the preceding description of the respective features of the combined ultrasonic and HF surgical system.

According to a further aspect of the invention there is provided a method for operating a combined ultrasonic and HF surgical system described above, comprising providing an excitation signal, by which an ultrasonic converter can generate an ultrasonic vibration, and providing an HF energy, characterized in that the provision of the excitation signal and the HF energy takes place in a coordinated fashion, such that the HF energy is provided in dependency of the excitation signal, wherein a HF energy is modulated with the frequency of the excitation signal or with a harmonic of that frequency.

The method according to the present invention and its enhancements have features or method steps, respectively, which make them in particular suitable to be used for a combined ultrasonic and HF surgical system according to the present invention and its enhancements. With respect to the advantages, embodiments and embodiment details of the method and its enhancements it is referred to the preceding description of the respective device features.

Preferred embodiments of the invention are described in an exemplary manner with reference to the attached figures, wherein
- Fig. 1:: illustrates a schematic illustration of an exemplary embodiment of a combined ultrasonic and HF surgical system;
- Fig. 2:: illustrates the dependency, with respect to time, between a position of an ultrasonic applicator and the provision of HF energy in different operating modes; and
- Fig. 3:: illustrates the dependency, with respect to time, between a position of an ultrasonic applicator and the provision of HF energy in further different operating modes.

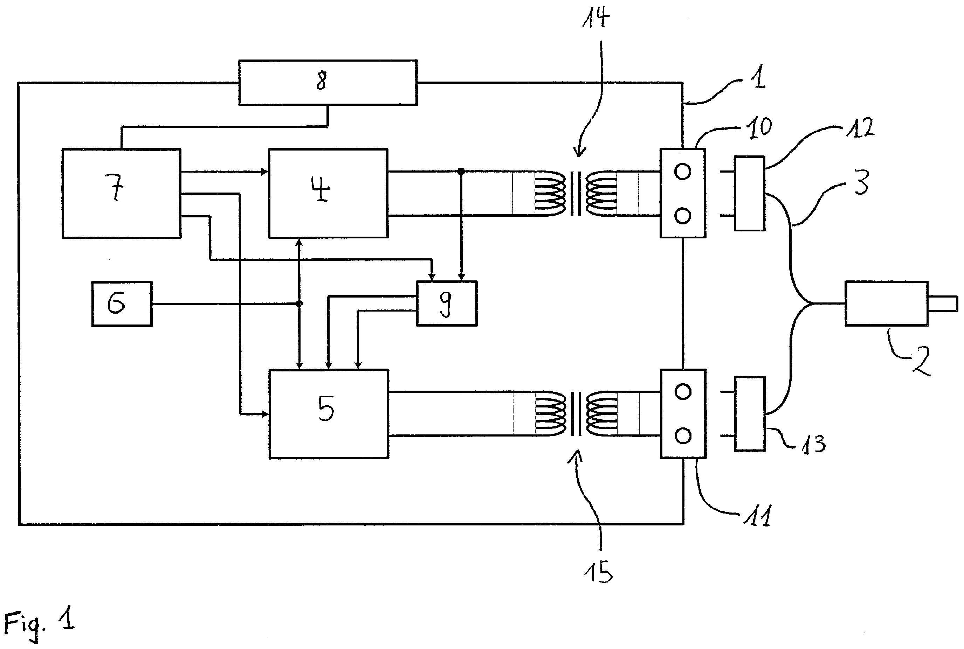

An exemplary combined ultrasonic and HF surgical system according to the invention is shown in Fig. 1. The system comprises an electrosurgical generator 1 and an electrosurgical instrument 2. Electrosurgical instrument 2 is connected to generator 1 by means of a cable 3. The electrosurgical instrument 2 may be the Thunderbeat forceps of Olympus Medical Systems Corporation, Tokyo, Japan, or any other instrument providing ultrasonic and radiofrequency treatment functionality.

The electrosurgical generator 1 comprises an ultrasonic frequency oscillator 4 for providing an excitation signal for an ultrasonic vibrator (not shown) in the electrosurgical instrument 2. The electrosurgical generator 1 further comprises a radiofrequency (HF) oscillator 5 for providing a radiofrequency treatment signal for a treatment electrode (not shown) in the electrosurgical instrument 2. The vibrator and/or electrode are of known design and need not further be explained.

Ultrasonic frequency oscillator 4 and radiofrequency oscillator 5 receive timing signals from a common clock 6. Separate clocks can also be provided for both oscillators 4, 5. Clock 6 may provide a timing signal of e.g. 1 kHz, and respective oscillators 4, 5 use the clock signal as a reference for producing output signals of desired frequencies, e.g. 20-50 kHz as ultrasonic frequency or 0.2-3 MHz as radiofrequency. Resonant feedback loops may also be used by oscillators 4, 5 instead of a fixed timing signal.

Main operational parameters of oscillators 4, 5 are set by a control unit 7 based on stored operational programs and/or user input through a graphical user interface 8.

The generator 1 further comprises a synchronization unit 9, which is connected to the control unit 7 as well as to both the ultrasonic frequency oscillator 4 and the radiofrequency oscillator 5. Synchronization unit 9 receives the excitation signal from the ultrasonic frequency oscillator 4 and uses this signal to generate one or more synchronized modulation signals which are output into radiofrequency oscillator 5.

A first synchronized modulation signal may be used to effect amplitude modulation of the radiofrequency treatment signal. A second synchronized modulation signal may be used to effect frequency modulation of the radiofrequency treatment signal. Synchronized modulation signals may be generated with the frequency of the ultrasonic frequency oscillator 4, or with a harmonic of that frequency, e.g. double or treble frequency, as controlled by the control unit 7, again based on stored operational programs and/or user input through user interface 8.

Modulation of the radiofrequency oscillator 5 may be effected by various techniques, such as modifying the activation time of energizing switches for feeding a resonant circuit for amplitude modulation, or by varying a capacity included in a resonant circuit for frequency modulation. Modulation may also be effected in the form of discrete switching between two operating frequencies or by cyclically switching the radiofrequency treatment signal on and off. Instead of switching the treatment signal off completely, it may be switched to a very low amplitude which has no therapeutic effect, but enables the radiofrequency oscillator 5 to maintain resonant operation.

Ultrasonic frequency excitation signal and radiofrequency treatment signal are fed to output sockets 10, 11 for connection of respective plugs 12,13 at the end of the cable 3 of the electrosurgical instrument 2. Transformers 14, 15 may be employed for galvanic separation of instrument 2 from generator 1.

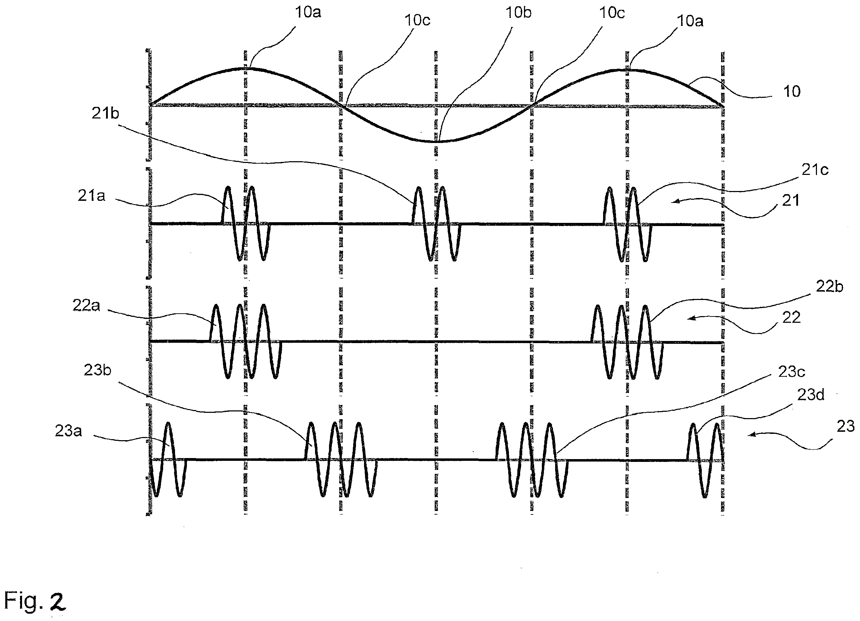

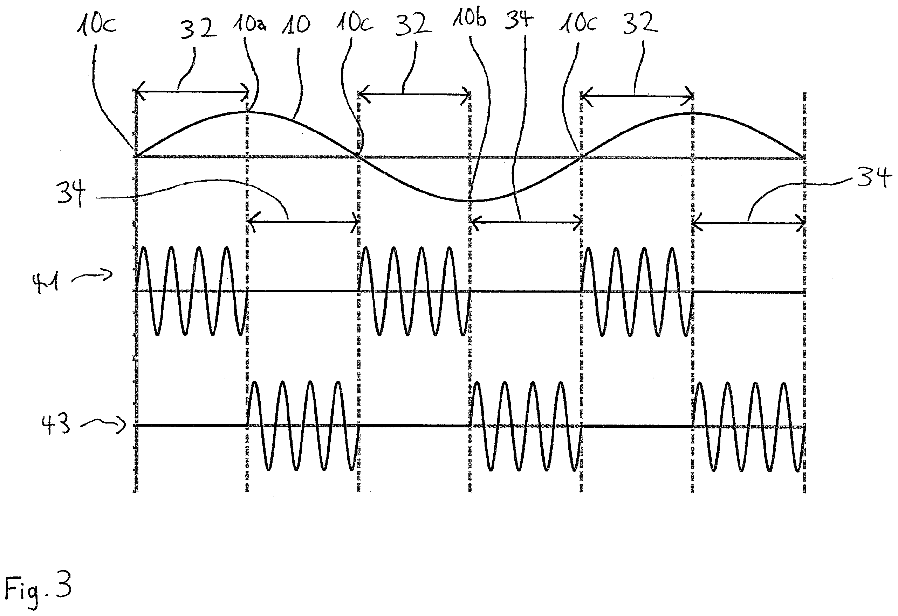

Various operational modes of the described system are explained below with reference to Figures 2 and 3.

Fig. 2 shows a diagram in which the time is indicated on the horizontal axis and four different curves are illustrated on top of each other on the vertical axis, which show the position of an ultrasonic applicator (top curve) as well as three different modes for providing HF energy (lower three curves).

The top curve labeled by 10 illustrates the position of an ultrasonic applicator of an instrument, the ultrasonic converter of which was excited by an excitation signal provided by an ultrasonic generator, and has generated from this excitation signal an ultrasonic vibration. Frequency and amplitude of this ultrasonic vibration of the ultrasonic applicator of the instrument can be seen at the top of Fig. 2 by curve 10. The curve has a number of turning points 10a, 10b, and zero crossings 10c. The turning points 10a are first turning points, the turning point 10b is a second turning point. The turning points 10a may correspond to maximum turning points of the excitation signal and the turning point 10b may correspond to a minimal turning point of the excitation.

The curves labeled by 21, 22, 23 show the provision of a HF energy in different dependency of the excitation signal or, respectively, the ultrasonic vibration, which can be seen at line 10 in Fig. 2.

Curve 21 shows an operating mode of a combined ultrasonic and HF surgical system, wherein the control unit is adapted to control the ultrasonic generator and/or the HF generator, such that HF energy 21 is provided at a time when an instrument, which is moved by the ultrasonic vibration, and/or the excitation signal is at turning points 10a, 10b. In Fig. 2 three intervals 21a, 21b, 21c of the provided HF energy 21 can be seen. As can be seen further in Fig. 2, the HF energy 21 is not only provided at the turning points 10a, 10b of curve 10, but also in a region around these turning points 10a, 10b. Between the phases of provision of HF energy 21a, 21b, 21c no HF energy is provided. Alternatively, HF energy can be provided with reduced amplitude and/or reduced frequency between the phases 21a, 21b, 21c.

Curve 23 shows an operating mode, which runs reverse compared to the operating mode illustrated by curve 21: According to curve 23, the control unit of a combined ultrasonic and HF surgical system is adapted to control the ultrasonic generator and/or the HF generator, such that HF energy 23 is provided at a time when the instrument, which is moved by the ultrasonic vibration, and/or the excitation signal lies between two turning points 10a, 10b, namely in a section of the zero crossings 10c, as labeled by 23a, 23b, 23c, 23d. Between the phases of providing of HF energy 23a, 23b, 23c, 23d no HF energy is provided according to curve 23, i.e. when the instrument, which is moved by the ultrasonic vibration, and/or the excitation signal lies in the region of the turning points 10a, 10b. Alternatively, HF energy can be provided with reduced amplitude and/or reduced frequency between the phases 23a, 23b, 23c, 23d.

A third operating mode is illustrated by curve 22. In this operating mode the control unit is adapted to control an ultrasonic generator and/or an HF generator such that HF energy 22 is provided at a time when the instrument, which is moved by the ultrasonic vibration, and/or the excitation signal is at a first turning point 10a or in a region of these first turning points 10a, respectively. According to curve 22, between the phases of provision of HF energy 22a, 22b, no HF energy is provided, i.e. in a region of a second turning point 10b and between two adjacent turning points (this means in a section of the zero crossings 10c) no HF energy is provided. Alternatively, HF energy can be provided with reduced amplitude and/or reduced frequency between the phases 22a, 22b.

By such a coordination of the provision of HF energy and the provision of ultrasonic energy, which results in a respective ultrasonic vibration of the instrument, synergetic effects between the two energies can be exploited in a better way. Furthermore, by the design of different operating modes the usage of combined ultrasonic and HF surgical systems can be adjusted to different fields of use and types of tissue.

Fig. 3 shows a diagram including further operating modes as compared to the diagram shown in Fig. 2. The same and similar features are indicated by the same reference numerals in Fig. 3. The time is indicated on the horizontal axis and three different curves are illustrated on top of each other on the vertical axis, which show the position of an ultrasonic applicator (top curve) as well as two different modes for providing HF energy (lower two curves).

As it is also shown in Fig. 2, the top curve labeled by 10 illustrates the position of an ultrasonic applicator of an instrument, the ultrasonic converter of which was excited by an excitation signal, provided by a generation unit of an ultrasonic generator, and has generated from this excitation signal an ultrasonic vibration. The curve is divided (double arrows) into two phases, namely a first phase 32 in which the moving velocity of the ultrasonic applicator decelerates and a second phase 34 in which the moving velocity of the ultrasonic applicator accelerates.

Curve 41 shows an operating mode of a combined ultrasonic and HF surgical system, wherein the control unit is adapted to control the ultrasonic generator and/or the HF generator, such that HF energy 41 is provided at a time 32 when an instrument, which is moved by the ultrasonic vibration, decelerates its moving velocity. The time when the instrument decelerates its moving velocity is the first phase 32 which starts at a zero crossing 10c and ends at a subsequent turning point 10a, 10b. The pressure exerted by the instrument while moving through the tissue is decreased during this first phase of decelaration. Therefore, the cutting ability is improved in the phase of deceleration.

Curve 43 shows an operating mode of a combined ultrasonic and HF surgical system, wherein the control unit is adapted to control the ultrasonic generator and/or the HF generator, such that HF energy 43 is provided at a time 34 when an instrument, which is moved by the ultrasonic vibration, accelerates its moving velocity. The time when the instrument accelerates its moving velocity is the second phase 34 which starts at a turning point 10a, 10b and ends half way to an adjacent turning point, namely at a zero crossing 10c. While moving through the tissue, the instrument exerts a pressure onto the tissue. During this second phase of acceleration, the pressure is increased. Therefore, the coagulating ability is improved in the phase.

As described above with regard to Figure 2, instead of shutting HF energy off between the phases 32 or 34, respectively, HF energy can be provided with reduced amplitude and/or reduced frequency between these phases.

## Claims

1. A control device for controlling a combined ultrasonic and HF surgical system comprising
- a control unit adapted to control an ultrasonic generator and/or a HF generator, such that an excitation signal, by which an ultrasonic converter can generate an ultrasonic vibration, and the HF energy are provided in a coordinated fashion, such that the HF energy is provided in dependency of the excitation signal, **characterised in that** the HF energy is modulated with the frequency of the excitation signal or with a harmonic of that frequency.

2. A combined ultrasonic and HF surgical system, comprising
- an ultrasonic generator having a generation unit for providing an excitation signal by which an ultrasonic converter can generate an ultrasonic vibration,
- a HF generator for providing a HF energy at two output contacts, and
- the control unit of claim 1.

3. A combined ultrasonic and HF surgical system according to claim 2,
**characterized in that** the control unit comprises a synchronization unit which is adapted to
- receive the excitation signal from the ultrasonic generator,
- generate at least one synchronized modulation signal in dependency of the excitation signal, and
- output the modulation signal to the HF generator.

4. A combined ultrasonic and HF surgical system according to any of the preceding claims 2-3, **characterized in that** the control unit is adapted to control the ultrasonic generator and/or the HF generator, such that a HF energy is modulated with a harmonic, in particular with the double or treble frequency of the excitation signal.

5. A combined ultrasonic and HF surgical system according to any of the preceding claims 2-4, **characterized in that** the control unit is adapted to control the ultrasonic generator and/or the HF generator, such that HF energy is provided with a first amplitude and/or with a first frequency at a time when an instrument, which is moved by the ultrasonic vibration, and/or the excitation signal is at a turning point, and that HF energy is provided with a second amplitude, which is smaller than the first amplitude, and/or with a second frequency, which is smaller than the first frequency, at a time when the instrument, which is moved by the ultrasonic vibration, and/or the excitation signal is between two turning points.

6. A combined ultrasonic and HF surgical system according to any of the preceding claims 2-5, **characterized in that** the control unit is adapted to control the ultrasonic generator and/or the HF generator, such that HF energy is provided with a first amplitude and/or with a first frequency at a time when an instrument, which is moved by the ultrasonic vibration, and/or the excitation signal is between two turning points, and such that HF energy is provided with a second amplitude, which is smaller than the first amplitude, and/or with a second frequency, which is smaller than the first frequency, at a time when the instrument, which is moved by the ultrasonic vibration, and/or the excitation signal is at a turning point.

7. A combined ultrasonic and HF surgical system, according to any of the preceding claims 2-6, **characterized in that** the control unit is adapted to control the ultrasonic generator and/or the HF generator, such that HF energy is provided with a first amplitude and/or with a first frequency at a time when an instrument, which is moved by the ultrasonic vibration, and/or the excitation signal is at a first turning point, and that HF energy is provided with a second amplitude, which is smaller than the first amplitude, and/or with a second frequency, which is smaller than the first frequency, at a time when the instrument, which is moved by the ultrasonic vibration, and/or the excitation signal is at a second turning point or between two turning points.

8. A combined ultrasonic and HF surgical system according to any of the preceding claims 2-7, **characterized in that** the control unit is adapted to control the ultrasonic generator and/or the HF generator, such that HF energy is provided with a first amplitude and/or with a first frequency at a time when an instrument, which is moved by the ultrasonic vibration, has no or a low moving velocity, and that HF energy is provided with a second amplitude, which is smaller than the first amplitude, and/or with a second frequency, which is smaller than the first frequency, at a time when the instrument, which is moved by the ultrasonic vibration, has a high moving velocity.

9. A combined ultrasonic and HF surgical system according to any of the preceding claims 2-8, **characterized in that** the control unit is adapted to control the ultrasonic generator and/or the HF generator, such that HF energy is provided with a first amplitude and/or with a first frequency at a time when an instrument, which is moved by the ultrasonic vibration, has a high moving velocity, and that HF energy is provided with a second amplitude, which is smaller than the first amplitude, and/or with a second frequency, which is smaller than the first frequency, at a time when the instrument, which is moved by the ultrasonic vibration, has a lower or no moving velocity.

10. A combined ultrasonic and HF surgical system according to any of the preceding claims 2-9, **characterized in that** the control unit is adapted to control the ultrasonic generator and/or the HF generator, such that HF energy is provided with a first amplitude and/or with a first frequency at a time when an instrument, which is moved by the ultrasonic vibration, accelerates its moving velocity, and that HF energy is provided with a second amplitude, which is smaller than the first amplitude, and/or with a second frequency, which is smaller than the first frequency, at a time when an instrument, which is moved by the ultrasonic vibration, decelerates its moving velocity.

11. A combined ultrasonic and HF surgical system according to any of the preceding claims 2-10, **characterized in that** the control unit is adapted to control the ultrasonic generator and/or the HF generator, such that HF energy is provided with a first amplitude and/or with a first frequency at a time when an instrument, which is moved by the ultrasonic vibration, decelerates its moving velocity, and that HF energy is provided with a second amplitude, which is smaller than the first amplitude, and/or with a second frequency, which is smaller than the first frequency, at a time when an instrument, which is moved by the ultrasonic vibration, accelerates its moving velocity.

12. A combined ultrasonic and HF surgical system according to any of the preceding claims 2-11, **characterized in that** the modulation of a HF energy is executed as an amplitude modulation by
- modifying the activation time of a switch provided for feeding a resonant circuit, or
- cyclically switching the HF energy substantially on and off.

13. A combined ultrasonic and HF surgical system according to any of the preceding claims 2-12, **characterized in that** the modulation of a HF energy is executed as a frequency modulation by
- varying a capacity included in a resonant circuit for frequency modulation, or
- switching between at least two operating frequencies.

## Patentansprüche

1. Steuervorrichtung zum Steuern eines kombinierten Ultraschall- und HF-Chirurgiesystems, umfassend
- eine Steuereinheit, die zum Steuern eines Ultraschallgenerators und/oder eines HF-Generators ausgelegt ist, sodass ein Anregungssignal, durch das ein Ultraschallwandler eine Ultraschallschwingung generieren kann, und die HF-Energie in koordinierter Weise bereitgestellt werden, sodass die HF-Energie in Abhängigkeit des Anregungssignals bereitgestellt wird, **dadurch gekennzeichnet, dass** die HF-Energie mit der Frequenz des Anregungssignals oder mit einer Oberschwingung dieser Frequenz moduliert ist.

2. Kombiniertes Ultraschall- und HF-Chirurgiesystem, umfassend
- einen Ultraschallgenerator mit einer Generierungseinheit zum Bereitstellen eines Anregungssignals, mit dem ein Ultraschallwandler eine Ultraschallschwingung generieren kann,
- einen HF-Generator zum Bereitstellen einer HF-Energie an zwei Ausgangskontakten und
- die Steuereinheit nach Anspruch 1.

3. Kombiniertes Ultraschall- und HF-Chirurgiesystem nach Anspruch 2, **dadurch gekennzeichnet, dass** die Steuereinheit eine Synchronisationseinheit umfasst, die ausgelegt ist um
- das Anregungssignal vom Ultraschallgenerator zu empfangen,
- mindestens ein synchronisiertes Modulationssignal in Abhängigkeit vom Anregungssignal zu generieren und
- das Modulationssignal an den HF-Generator auszugeben.

4. Kombiniertes Ultraschall- und HF-Chirurgiesystem nach einem der vorstehenden Ansprüche 2-3, **dadurch gekennzeichnet, dass** die Steuereinheit ausgelegt ist, um den Ultraschallgenerator und/oder den HF-Generator so zu steuern, dass eine HF-Energie mit einer Oberschwingung, insbesondere mit der Doppel- oder Dreifachfrequenz des Anregungssignals, moduliert wird.

5. Kombiniertes Ultraschall- und HF-Chirurgiesystem nach einem der vorhergehenden Ansprüche 2-4, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, und/oder das Anregungssignal sich an einem Wendepunkt befindet, und dass HF-Energie mit einer zweiten Amplitude, die kleiner als die erste Amplitude ist, und/oder mit einer zweiten Frequenz, die kleiner als die erste Frequenz ist, zu einer Zeit bereitgestellt wird, zu der sich das Instrument, das durch die Ultraschallschwingung bewegt wird, und/oder das Anregungssignal zwischen zwei Wendepunkten befindet.

6. Kombiniertes Ultraschall- und HF-Chirurgiesystem nach einem der vorhergehenden Ansprüche 2-5, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, und/oder das Anregungssignal sich zwischen zwei Wendepunkten befindet, und derart, dass HF-Energie mit einer zweiten Amplitude, die kleiner als die erste Amplitude ist, und/oder mit einer zweiten Frequenz, die kleiner als die erste Frequenz ist, zu einer Zeit bereitgestellt wird, zu der das durch die Ultraschallschwingung bewegte Instrument und/oder das Anregungssignal sich an einem Wendepunkt befindet.

7. Kombiniertes Ultraschall- und HF-Chirurgiesystem nach einem der vorhergehenden Ansprüche 2-6, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, und/oder das Anregungssignal sich an einem ersten Wendepunkt befindet, und dass HF-Energie mit einer zweiten Amplitude, die kleiner als die erste Amplitude ist, und/oder mit einer zweiten Frequenz, die kleiner als die erste Frequenz ist, zu einer Zeit bereitgestellt wird, zu der sich das Instrument, das durch die Ultraschallschwingung bewegt wird, und/oder das Anregungssignal an einem zweiten Wendepunkt oder zwischen zwei Wendepunkten befindet.

8. Kombiniertes Ultraschall- und HF-Chirurgiesystem nach einem der vorhergehenden Ansprüche 2-7, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, keine oder eine geringe Bewegungsgeschwindigkeit aufweist, und dass HF-Energie mit einer zweiten Amplitude, die kleiner als die erste Amplitude ist, und/oder mit einer zweiten Frequenz, die kleiner als die erste Frequenz ist, zu einer Zeit bereitgestellt ist, zu der das Instrument, das durch die Ultraschallschwingung bewegt wird, eine hohe Bewegungsgeschwindigkeit aufweist.

9. Kombiniertes Ultraschall- und HF-Chirurgiesystem nach einem der vorhergehenden Ansprüche 2-8, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, eine hohe Bewegungsgeschwindigkeit aufweist, und dass HF-Energie mit einer zweiten Amplitude, die kleiner als die erste Amplitude ist, und/oder mit einer zweiten Frequenz, die kleiner als die erste Frequenz ist, zu einer Zeit bereitgestellt ist, zu der das Instrument, das durch die Ultraschallschwingung bewegt wird, eine geringere oder keine Bewegungsgeschwindigkeit aufweist.

10. Kombiniertes Ultraschall- und HF-Chirurgiesystem nach einem der vorhergehenden Ansprüche 2-9, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, seine Bewegungsgeschwindigkeit beschleunigt, und dass HF-Energie mit einer zweiten Amplitude, die kleiner als die erste Amplitude ist, und/oder mit einer zweiten Frequenz, die kleiner als die erste Frequenz ist, zu einer Zeit bereitgestellt wird, zu der ein Instrument, das durch die Ultraschallschwingung bewegt wird, seine Bewegungsgeschwindigkeit verringert.

11. Kombiniertes Ultraschall- und HF-Chirurgiesystem nach einem der vorstehenden Ansprüche 2-10, **dadurch gekennzeichnet, dass** die Steuereinheit ausgelegt ist, um den Ultraschallgenerator und/oder den HF-Generator so zu steuern, dass HF-Energie mit einer ersten Amplitude und/oder mit einer ersten Frequenz zu einer Zeit bereitgestellt wird, zu der ein Instrument, das durch die Ultraschallschwingung bewegt wird, seine Bewegungsgeschwindigkeit verringert, und dass HF-Energie mit einer zweiten Amplitude, die kleiner als die erste Amplitude ist, und/oder mit einer zweiten Frequenz, die kleiner als die erste Frequenz ist, zu einer Zeit bereitgestellt wird, zu der ein Instrument, das durch die Ultraschallschwingung bewegt wird, seine Bewegungsgeschwindigkeit beschleunigt.

12. Kombiniertes Ultraschall- und HF-Chirurgiesystem nach einem der vorhergehenden Ansprüche 2-11, **dadurch gekennzeichnet, dass** die Modulation einer HF-Energie als Amplitudenmodulation ausgeführt wird durch
- Modifizieren der Aktivierungszeit eines Schalters, der zum Speisen eines Resonanzkreises bereitgestellt ist, oder
- im Wesentlichen zyklisches Ein- und Ausschalten der HF-Energie.

13. Kombiniertes Ultraschall- und HF-Chirurgiesystem nach einem der vorhergehenden Ansprüche 2-12, **dadurch gekennzeichnet, dass** die Modulation einer HF-Energie als Frequenzmodulation ausgeführt wird durch
- Variieren einer Kapazität, die in einem Resonanzkreis zur Frequenzmodulation enthalten ist, oder
- Schalten zwischen mindestens zwei Betriebsfrequenzen.

## Revendications

1. Dispositif de commande pour commander un système ultrasonore et HF chirurgical combiné, comprenant
- une unité de commande adaptée pour commander un générateur ultrasonore et/ou un générateur HF, de telle manière qu'un signal d'excitation, par lequel un convertisseur à ultrasons peut générer une vibration ultrasonore, et l'énergie HF sont fournis d'une manière coordonnée, de telle manière que l'énergie HF est fournie en fonction du signal d'excitation, **caractérisé en ce que** l'énergie HF est modulée avec la fréquence du signal d'excitation ou avec une harmonique de cette fréquence.

2. Système ultrasonore et HF chirurgical combiné, comprenant
- un générateur ultrasonore comportant une unité de génération pour fournir un signal d'excitation par lequel un convertisseur à ultrasons peut générer une vibration ultrasonore,
- un générateur HF pour fournir une énergie HF au niveau de deux contacts de sortie, et
- l'unité de commande selon la revendication 1.

3. Système ultrasonore et HF chirurgical combiné selon la revendication 2, **caractérisé en ce que** l'unité de commande comprend une unité de synchronisation qui est adaptée pour
- recevoir le signal d'excitation à partir du générateur ultrasonore,
- générer au moins un signal de modulation synchronisé en fonction du signal d'excitation, et
- délivrer en sortie le signal de modulation au générateur HF.

4. Système ultrasonore et HF chirurgical combiné selon l'une quelconque des revendications précédentes 2 et 3, **caractérisé en ce que** l'unité de commande est adaptée pour commander le générateur ultrasonore et/ou le générateur HF, de telle manière qu'une énergie HF est modulée avec une harmonique, en particulier avec la double ou la triple fréquence du signal d'excitation.

5. Système ultrasonore et HF chirurgical combiné selon l'une quelconque des revendications précédentes 2 à 4, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, et/ou le signal d'excitation est à un tournant, et **en ce qu'**une énergie HF est fournie ayant une seconde amplitude, qui est inférieure à la première amplitude, et/ou ayant une seconde fréquence, qui est inférieure à la première fréquence, à un moment où l'instrument, qui est déplacé par la vibration ultrasonore, et/ou le signal d'excitation est entre deux tournants.

6. Système ultrasonore et HF chirurgical combiné selon l'une quelconque des revendications précédentes 2 à 5, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, et/ou le signal d'excitation est entre deux tournants, et de telle manière qu'une énergie HF est fournie ayant une seconde amplitude, qui est inférieure à la première amplitude, et/ou ayant une seconde fréquence, qui est inférieure à la première fréquence, à un moment où l'instrument, qui est déplacé par la vibration ultrasonore, et/ou le signal d'excitation est à un tournant.

7. Système ultrasonore et HF chirurgical combiné, selon l'une quelconque des revendications précédentes 2 à 6, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, et/ou le signal d'excitation est à un premier tournant, et **en ce qu'**une énergie HF est fournie ayant une seconde amplitude, qui est inférieure à la première amplitude, et/ou ayant une seconde fréquence, qui est inférieure à la première fréquence, à un moment où l'instrument, qui est déplacé par la vibration ultrasonore, et/ou le signal d'excitation est à un second tournant ou entre deux tournants.

8. Système ultrasonore et HF chirurgical combiné selon l'une quelconque des revendications précédentes 2 à 7, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, a une vitesse de déplacement nulle ou faible, et **en ce qu'**une énergie HF est fournie ayant une seconde amplitude, qui est inférieure à la première amplitude, et/ou ayant une seconde fréquence, qui est inférieure à la première fréquence, à un moment où l'instrument, qui est déplacé par la vibration ultrasonore, a une vitesse de déplacement élevée.

9. Système ultrasonore et HF chirurgical combiné selon l'une quelconque des revendications précédentes 2 à 8, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, a une vitesse de déplacement élevée, et **en ce qu'**une énergie HF est fournie ayant une seconde amplitude, qui est inférieure à la première amplitude, et/ou ayant une seconde fréquence, qui est inférieure à la première fréquence, à un moment où l'instrument, qui est déplacé par la vibration ultrasonore, a une vitesse de déplacement inférieure ou nulle.

10. Système ultrasonore et HF chirurgical combiné selon l'une quelconque des revendications précédentes 2 à 9, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, accélère sa vitesse de déplacement, et **en ce qu'**une énergie HF est fournie ayant une seconde amplitude, qui est inférieure à la première amplitude, et/ou ayant une seconde fréquence, qui est inférieure à la première fréquence, à un moment où un instrument, qui est déplacé par la vibration ultrasonore, décélère sa vitesse de déplacement.

11. Système ultrasonore et HF chirurgical combiné selon l'une quelconque des revendications précédentes 2 à 10, **caractérisé en ce que** l'unité de commande est adaptée pour commander le générateur ultrasonore et/ou le générateur HF, de telle manière qu'une énergie HF est fournie ayant une première amplitude et/ou ayant une première fréquence à un moment où un instrument, qui est déplacé par la vibration ultrasonore, décélère sa vitesse de déplacement, et **en ce qu'**une énergie HF est fournie ayant une seconde amplitude, qui est inférieure à la première amplitude, et/ou ayant une seconde fréquence, qui est inférieure à la première fréquence, à un moment où un instrument, qui est déplacé par la vibration ultrasonore, accélère sa vitesse de déplacement.

12. Système ultrasonore et HF chirurgical combiné selon l'une quelconque des revendications précédentes 2 à 11, **caractérisé en ce que** la modulation d'une énergie HF est exécutée en tant que modulation d'amplitude par
- modification du temps d'activation d'un commutateur fourni pour alimenter un circuit résonnant, ou
- commutation cyclique de l'énergie HF sensiblement en marche et à l'arrêt.

13. Système ultrasonore et HF chirurgical combiné selon l'une quelconque des revendications précédentes 2 à 12, **caractérisé en ce que** la modulation d'une énergie HF est exécutée tant que modulation de fréquence par
- variation d'une capacité incluse dans un circuit résonnant pour modulation de fréquence, ou
- commutation entre au moins deux fréquences de fonctionnement.
